(19) Europäisches Patentamt

European Patent Office

Office européen des brevets

(11) EP 4 769 427 A1

(12) **EUROPEAN PATENT APPLICATION**

(43) Date of publication:
01.07.2026 Bulletin 2026/27

(21) Application number: 25227423.8

(22) Date of filing: 29.12.2025

(51) International Patent Classification (IPC):
$G16H\ 40/40^{(2018.01)}$    $G16H\ 40/63^{(2018.01)}$

(52) Cooperative Patent Classification (CPC):
G16H 40/63; G16H 40/40

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 27.12.2024 CN 202411954216
31.12.2024 CN 202411992249

(71) Applicants:
• Jiangsu Yuwell POCTech Biotechnology Co.,
Ltd.
Zhenjiang, Jiangsu 212300 (CN)

• Jiangsu Yuekai Biotechnology Co., Ltd.
Nanjing, Jiangsu 210018 (CN)
• Zhejiang Poctech Co., Ltd.
Huzhou, Zhejiang 313001 (CN)

(72) Inventors:
• Guan, Zhongda
Nanjing, Jiangsu, 210018 (CN)
• Liang, Zhen
Nanjing, Jiangsu, 210018 (CN)
• Zhang, Guanqun
Nanjing, Jiangsu, 210018 (CN)

(74) Representative: Winter, Brandl - Partnerschaft
mbB
Alois-Steinecker-Straße 22
85354 Freising (DE)

(54) **METHOD OF DETECTING AN ANOMALY OF A SIGNAL FROM A PHYSIOLOGICAL PARAMETER DETECTION APPARATUS, DEVICE AND MEDIUM**

(57) This application discloses a method and device for detecting an anomaly of a signal from a physiological parameter detection apparatus, particularly a continuous glucose monitor, CGM, comprising: acquiring a working parameter of the physiological parameter detection apparatus, wherein the working parameter comprises a working current; determining whether the signal from the physiological parameter detection apparatus is abnormal based on the working parameter: and outputting a detection result. Through the foregoing solution, an anomaly of a signal from the physiological parameter detection apparatus can be effectively detected, thereby ensuring signal accuracy.

S1 → acquiring a working parameter of the physiological parameter detection apparatus, wherein the working parameter comprises a working current

S2 → based on the working parameter, determining whether the signal from the physiological parameter detection apparatus is abnormal

S3 → outputting a detection result

FIG. 1

EP 4 769 427 A1

## Description

### TECHNICAL FIELD

This application relates to the field of signal detection, and specifically, to a method and device for detecting an anomaly of a signal from a physiological parameter detection apparatus, and a medium.

### BACKGROUND

With the development of medical technologies, physiological parameter detection apparatuses such as continuous glucose monitoring system have been used more widely. However, at present, some physiological parameter detection apparatuses are likely to become faulty and causes a signal anomaly due to problems such as noise, sensor performance drift, breakage of a screen-printed electrode, compression during wearing, and short circuit caused by blood. Because faults of such an apparatus are directly associated with accuracy and stability of measurement of physiological parameters, a detection problem of a signal anomaly (fault detection problem) for physiological parameter detection apparatuses is becoming increasingly prominent.

For example, various types of noise existing in an environment, such as electromagnetic interference and mechanical vibration noise, may be superimposed on the signals acquired by the physiological parameter detection apparatus, causing distortion and deformation in original pure signals. Alternatively, as use time of a sensor increases, the sensitivity may degrade. As a result, the acquired signals deviate from true values. Alternatively, if a sensor is not correctly worn and has a displacement or the like, the acquired signals may be inaccurate. In this case, once the obtained untrue human vital sign signals are used as valid data, a medical worker may be misled to make an improper treatment decision. For example, a drug dose may be incorrectly adjusted, or a treatment solution may be changed. Not only a disease cannot be effectively treated, but also the condition of the patient may even be worsened.

Error code detection means that when human vital sign signals (physiological parameter signals) are detected through sensors, obtained human vital sign signals are not actual signals due to the impact of problems such as noise, sensor performance, and device wearing. Detecting these error signals and outputting corresponding error codes are ErrorCode detection.

Faults of the physiological parameter detection apparatus may be classified into two types: hard faults and soft faults. Hard faults mainly mean that the apparatus itself or a sensor thereof is faulty, such as cracks in the sensor's soft needle, and a working current detected by the apparatus having a hard fault is an invalid signal. Soft faults are mostly faults that can be corrected (are recoverable) and manifest as abnormal operating currents or invalid signals. However, soft faults are not problems with the sensor or apparatus itself, but are mainly generated during the use of the apparatus, such as poor contact caused by squeezing or improper latching. These faults can be eliminated through manual intervention or other corrective measure.

Fault monitoring of an existing physiological parameter detection apparatuses generally relies on manual checking and judgement based on experience, which is inefficient and susceptible to human factors. Another method is to provide an additional monitoring device or sensor to achieve redundancy, so that when a fault occurs in a device, the fault can be detected by comparing a measurement result of the device with a measurement result of another device. However, this method increases the economic load on patients and the risk of physical harm to patients.

### SUMMARY

To solve the above problems, this application provides a method of detecting an anomaly of a signal from a physiological parameter detection apparatus, comprising:

acquiring a working parameter of the physiological parameter detection apparatus, wherein the working parameter comprises a working current;
determining whether the signal from the physiological parameter detection apparatus is abnormal based on the working parameter, and
outputting a detection result.

Optionally, said signal is a human vital sign signal, and the human vital sign signal is calculated from the working current. Optionally, the method (particularly for detecting an error code in a human vital sign signal), comprises:

generating a first state vector based on the human vital sign signal and a preset signal value;
determining an estimated vital sign signal at a current moment based on the human vital sign signal at a previous moment through a filtering model; determining a predicted signal interval at the current moment based on the estimated vital sign signal and the human vital sign signal within a preset time window through a constraint model; generating a second state vector based on the estimated vital sign signal and the predicted signal interval; and determining, based on the first state vector and the second state vector, whether an error code exists in a human vital sign signal at the current moment, and outputting the detection result.

Optionally, the determining an estimated vital sign signal at a current moment based on a human vital sign signal at a previous moment through a filtering model comprises:

linearizing a state equation of the filtering model to obtain a linear state equation; and
obtaining the estimated vital sign signal at the current moment based on an optimal estimation result of the human vital sign signal at the previous moment by using the linear state equation.

Optionally, the determining a predicted signal interval at the current moment based on the estimated vital sign signal and a human vital sign signal within a preset time window through a constraint model includes:

calculating a signal standard deviation and a signal mean based on the human vital sign signal within the preset time window; and
inputting the signal standard deviation, the signal mean, the human vital sign signal at the previous moment, and the estimated vital sign signal to the constraint model, and determining, by the constraint model, the predicted signal interval at the current moment.

Optionally, the predicted signal interval at the current moment comprises a maximum critical value and a minimum critical value;

a calculation formula of the maximum critical value is:

$$\text{upLimit\_CgmSignal}_i$$
$$= G(\text{Pro\_CgmSignal}_{i-1}, \text{Ave\_Pro\_CgmSignal}_N, \bar{x}_{k+1|k}) + \gamma$$
$$* \text{Std\_Pro\_CgmSignal}_N$$

a calculation formula of the minimum critical value is:

$$\text{LowLimit\_CgmSignal}_i$$
$$= G(\text{Pro\_CgmSignal}_{i-1}, \text{Ave\_Pro\_CgmSignal}_N, \bar{x}_{k+1|k}) - \beta$$
$$* \text{Std\_Pro\_CgmSignal}_N$$

$\text{Pro\_CgmSignal}_{i-1}$ represents the human vital sign signal at the previous moment; $\text{Ave\_Pro\_CgmSignal}_N$ represents the signal mean; $\text{Std\_Pro\_CgmSignal}_N$ represents the signal standard deviation; $\bar{x}_{k+1|k}$ represents the estimated vital sign signal at the current moment; and $\gamma$ and $\beta$ represent preset coefficients.

Optionally, the preset signal value comprises a first signal value, a second signal value, and a third signal value; before the generating a first state vector based on a human vital sign signal and a preset signal value, the method further includes:

preprocessing the human vital sign signal based on the first signal value, the second signal value, and the third signal value, to obtain a preprocessed vital sign update signal; and
correspondingly, the generating a first state vector based on a human vital sign signal and a preset signal value comprises:
generating the first state vector based on the vital sign update signal.

Optionally, the preprocessing the human vital sign signal based on the first signal value, the second signal value, and the third signal value comprises:

updating the human vital sign signal to the first signal value if the human vital sign signal is greater than the first signal value;

updating the human vital sign signal to a sum value of the human vital sign signal at the previous moment and the second signal value if the human vital sign signal is greater than a human vital sign signal at the previous moment and a difference between the human vital sign signal and the human vital sign signal at the previous moment is greater than the second signal value; and

updating the human vital sign signal to a difference between the human vital sign signal at the previous moment and the third signal value if the human vital sign signal is less than the human vital sign signal at the previous moment and an absolute value of the difference between the human vital sign signal and the human vital sign signal at the previous moment is greater than the third signal value.

Optionally, the generating the first state vector based on the vital sign update signal (determined by the first signal value, the second signal value, and the third signal value) comprises:

determining a state value corresponding to the vital sign update signal as a first preset value if the human vital sign signal is greater than or equal to the first signal value or if the human vital sign signal is 0;

determining the state value corresponding to the vital sign update signal as the first preset value if the human vital sign signal is greater than a human vital sign signal at the previous moment and an absolute value of the difference between the human vital sign signal and the human vital sign signal at the previous moment is greater than or equal to the second signal value;

determining the state value corresponding to the vital sign update signal as the first preset value if the human vital sign signal is less than a human vital sign signal at the previous moment and the absolute value of the difference between the human vital sign signal and the human vital sign signal at the previous moment is greater than or equal to the third signal value;

determining the state value corresponding to the vital sign update signal as a second preset value if the human vital sign signal is not less than a human vital sign signal at the previous moment, or the absolute value of the difference between the human vital sign signal and the human vital sign signal at the previous moment is less than the third signal value; and

generating the first state vector based on the state value of each vital sign update signal.

Optionally, the generating a second state vector based on the estimated vital sign signal and the predicted signal interval comprises:

determining a state value corresponding to the estimated vital sign signal as a second preset value if the estimated vital sign signal falls within the predicted signal interval;

determining the state value corresponding to the estimated vital sign signal as a first preset value if the estimated vital sign signal does not fall within the predicted signal interval; and

generating the second state vector based on the state value of each estimated vital sign signal.

Optionally, the determining, based on the first state vector and the second state vector, whether an error code exists in a human vital sign signal at the current moment comprises:

if a state value of the first state vector corresponding to the estimated vital sign signal is a first preset value or a state value of the second state vector is the first preset value, determining that an error code exists in the human vital sign signal at the current moment; and

if the state value of the first state vector corresponding to the estimated vital sign signal is a second preset value and the state value of the second state vector is the second preset value, determining that the human vital sign signal at the current moment is a valid code.

Optionally, after the determining, based on the first state vector and the second state vector, whether an error code exists in a human vital sign signal at the current moment, the method further comprises:

determining an optimal estimation result corresponding to the human vital sign signal at the current moment, and acquiring a number of instances of consecutive occurrences of determining that the human vital sign signal at the current moment is a valid code;

outputting the human vital sign signal at the current moment as an unrepairable signal if the number of consecutive occurrences is greater than or equal to a third preset value; and

outputting the human vital sign signal at the current moment as a repairable signal if the number of consecutive occurrences is less than the third preset value.

Optionally, the working parameter further comprises a working voltage.

Further, optionally, the working voltage comprises a real-time working voltage, and the working current comprises a real-time working current. The method (particularly a method for detecting a fault type a physiological parameter detection apparatus) further comprises:

comparing the real-time working voltage with a first preset threshold to obtain a voltage determination result, and comparing the real-time working current with a second preset threshold to obtain a current determination result; and determining whether the signal is abnormal according to the voltage determination result and the current determination result, wherein the signal is the real-time working current.

In the present application, the term "real-time" refers to an operation of real-time systems that can reliably deliver specific results within a predetermined period of time, for example, in a fixed or variable time interval.

Optionally, the method further comprises: determining a fault type of the physiological parameter detection apparatus according to the voltage determination result and the current determination result.

In a feasible embodiment, the real-time working voltage includes a working electrode voltage, a reference electrode voltage, and a counter electrode voltage, the first preset threshold includes a first preset voltage threshold and a second preset voltage threshold, and the comparing the real-time working voltage with a first preset threshold to determine a voltage determination result comprises:

comparing the working electrode voltage with the first preset voltage threshold, and comparing the reference electrode voltage with the second preset voltage threshold, to determine a first voltage determination result; determining a second voltage determination result based on the working electrode voltage, the reference electrode voltage, and the counter electrode voltage; and determining that the voltage determination result indicates an abnormality if there is an abnormal result in the first voltage determination result or the second voltage determination result.

In a feasible embodiment, the first preset threshold further includes a first preset voltage difference threshold and a second preset voltage difference threshold, and the determining a second voltage determination result based on the working electrode voltage, the reference electrode voltage, and the counter electrode voltage comprises:

determining a first voltage difference between the working electrode voltage and the reference electrode voltage, and a second voltage difference between the working electrode voltage and the counter electrode voltage; and determining that the second voltage determination result indicates an abnormality if the first voltage difference is greater than the first preset voltage difference threshold or the second voltage difference is greater than the second preset voltage difference threshold.

In a feasible embodiment, the comparing the real-time working current with a second preset threshold to determine a current determination result comprises:

obtaining a first scope value corresponding to detection sensitivity of the physiological parameter detection apparatus, and obtaining a second scope value corresponding to a to-be-detected physiological parameter; determining a threshold interval of the real-time working current according to the first scope value and the second scope value, and determining the real-time working current based on the threshold interval, to obtain a first current determination result; performing signal filtering processing on the real-time working current, and determining a second current determination result according to the processed real-time working current and a preset current threshold; performing low-pass filtering processing on the real-time working current to obtain a baseline of the real-time working current, and determining a third current determination result based on drift data of the baseline and a preset drift data threshold corresponding to the baseline; and determining that the current determination result is an abnormality if there is an abnormal result in the first current determination result, the second current determination result, or the third current determination result.

In a feasible embodiment, the fault type includes a soft fault and a hard fault, and the determining a fault type of the physiological parameter detection apparatus according to the voltage determination result and the current determination result comprises:

determining that the fault type is the hard fault if the voltage determination result is an abnormality;

determining that the fault type is the hard fault if the third current determination result is an abnormality; and detecting whether the abnormality is corrected within a preset time step if the first current determination result or the second current determination result is an abnormality; determining that the fault type is the soft fault if the abnormality is corrected within the preset time step; and determining that the fault type is the hard fault if the abnormality is not corrected within the preset time step.

In a feasible embodiment, after the determining a fault type of the physiological parameter detection apparatus, the method further comprises:
counting a number of times the physiological parameter detection apparatus has a hard fault, and outputting, by the physiological parameter detection apparatus, an alarm signal if the counted number of times exceeds a preset number threshold.

In a feasible embodiment, before the obtaining a real-time working voltage and a real-time working current of the physiological parameter detection apparatus, the method further comprises:

collecting an initial voltage of the physiological parameter detection apparatus according to a preset sampling period; calculating an average voltage, a standard deviation, and a range value that correspond to the initial voltage, and determining whether the average voltage, the standard deviation, and the range value are all within corresponding preset thresholds; and
if the average voltage, the standard deviation, and the range value are all within the corresponding preset thresholds, obtaining the real-time working voltage and the real-time working current of the physiological parameter detection apparatus.

In a feasible embodiment, the method further comprises:
determining that the real-time working current is an invalid signal if the voltage determination result or the current determination result is an abnormality.

In a feasible embodiment, the method comprises:

determining whether the working parameter includes a real-time working voltage;
based on a determination that the working parameter includes the real-time working voltage, implementing a current-and-voltage-based anomaly detection process; and
based on a determination that the working parameter does not include the real-time working voltage, implementing a current-based anomaly detection process.

Preferably, the current-and-voltage-based anomaly detection process comprises steps of the method for detecting a fault type of a physiological parameter detection apparatus and the current-based anomaly detection process comprises steps of the method for detecting an error code in a human vital sign signal.

In another aspect, this application further provides a device for detecting an anomaly of a signal from a physiological parameter detection apparatus (particularly a device for detecting an error code in a human vital sign signal or a device for detecting a fault type of a physiological parameter detection apparatus), comprising:

at least one processor; and
a memory communicatively connected to the at least one processor,
where the memory has instructions that are executable by the at least one processor stored therein, and the instructions are executed by the at least one processor to cause the at least one processor to perform the method of detecting an anomaly of a signal from a physiological parameter detection apparatus (particularly a method for detecting the error code in the human vital sign signal or a method for detecting a fault type of a physiological parameter detection apparatus) described in any one of the above examples.

In another aspect, this application further provides a non-volatile computer storage medium, having computer-executable instructions stored therein. The computer-executable instructions are configured to implement the method of detecting an anomaly of a signal from a physiological parameter detection apparatus (particularly a method for detecting the error code in the human vital sign signal or a method for detecting a fault type of a physiological parameter detection apparatus) described in any one of the above examples.

The present application further provides physiological parameter detection apparatus comprising the device for detecting an anomaly of a signal from a physiological parameter detection apparatus described in any one of the above examples.

The method of detecting an anomaly of a signal from a physiological parameter detection apparatus (particularly a method for detecting the error code in the human vital sign signal or a method for detecting a fault type of a physiological parameter detection apparatus) provided in this application can bring the following beneficial effects:

This application can accurately detect changes of the signal in the physiological parameter detection apparatus and quickly identify abnormal signal conditions by monitoring a working parameter of the physiological parameter detection apparatus.

In an embodiment of this application, the first state vector based on the human vital sign signal and the preset signal value, and the second state vector based on the estimated vital sign signal and the predicted signal interval are constructed, so that a change trend and an expected range of the human vital sign signal can be comprehensively considered, thereby more accurately identifying an abnormal or error code and improving signal accuracy. In addition, the filtering model is used to predict a vital sign signal at the current moment, and the predicted signal interval is determined by using the constraint model, so that a human vital sign signal can be estimated and detected in real time without relying on a large amount of historical data, thereby improving timeliness of error code detection.

In an embodiment of this application, by monitoring a real-time working voltage and a real-time working current of a physiological parameter detection apparatus in real time, a change of a working signal of the apparatus can be quickly captured, and a timely information source is provided for fault detection, so that a fault can be detected in an early stage, and continuity and stability of a device can be ensured for a physiological parameter detection apparatus that needs to continuously run. In addition, the real-time working voltage and the real-time working current are respectively compared with corresponding thresholds to obtain the voltage determination result and the current determination result, and comprehensive analysis is performed based on the two determination results, to further determine a specific type of a fault. Compared with a conventional manual detection manner, an error rate of a manual operation can be reduced, and a fault type existing in the physiological parameter detection apparatus can be quickly and accurately reflected, thereby preventing an invalid signal collected by a faulty apparatus from being continuously used, and improving reliability of a detection signal of the physiological parameter detection apparatus. In addition, determination of the fault type can provide targeted guidance for subsequent fault correction, thereby improving maintenance efficiency.

**BRIEF DESCRIPTION OF THE DRAWINGS**

The accompanying drawings described herein are used to provide a further understanding of this application, and form part of this application. Exemplary embodiments of this application and descriptions thereof are used to explain this application, and do not constitute any inappropriate limitation to this application. In the accompanying drawings:

FIG. 1 is a schematic diagram of a method of detecting an anomaly of a signal from a physiological parameter detection apparatus according to an embodiment of this application;

FIG. 2 is a schematic flowchart of a first embodiment in embodiments of this application (particularly a method for detecting an error code in a human vital sign signal);

FIG. 3 is a schematic diagram of the first embodiment in embodiments of this application (particularly a method for detecting an error code in a human vital sign signal) in a specific case;

Fig. 4 is a schematic flowchart of a second embodiment in embodiments of this application (particularly a method for detecting a fault type of a physiological parameter detection apparatus);

FIG. 5 is a schematic diagram of a working circuit of a physiological parameter detection apparatus in an application scenario according to an embodiment of this application;

FIG. 6 is a schematic diagram of a logical framework of fault type detection of a physiological parameter detection apparatus according to the second embodiment (particularly the fault type detection) in embodiments this application;

FIG. 7 is a schematic diagram of a method of detecting an anomaly of a signal from a physiological parameter detection apparatus according to another specific embodiment in embodiments of this application;

FIG. 8 is a schematic diagram of a device for detecting an anomaly of a signal from a physiological parameter detection apparatus (particularly a device for detecting an error code in a human vital sign signal or a device for detecting a fault type of a physiological parameter detection apparatus) according to an embodiment of this application.

**DETAILED DESCRIPTION**

To clearly state the objectives, technical solutions, and advantages of this application, the technical solutions of this application will be clearly and completely described below with reference to specific embodiments of this application and the accompanying drawings. Apparently, the described embodiments are only some embodiments rather than all the embodiments of this application.

The following describes the technical solution provided in each embodiment of this application in detail with reference to the accompanying drawings. To understand the foregoing objectives, features, and advantages of this application more clearly, the following further describes this application in detail with reference to the accompanying drawings and specific implementations. It is to be noted that embodiments of this application and features in the embodiments may be combined without a conflict.

In the following description, many specific details are described to make this application fully understood. However, this application may alternatively be implemented in other manners different from those described herein. Therefore, the protection scope of this application is not limited to the specific embodiments disclosed below.

In addition, in the description of this application, it is to be understood that terms "first" and "second" are merely used for the purpose of description, and cannot be understood as indicating or implying relative importance or implicitly specifying the number of indicated technical features. Therefore, a feature defined by "first" or "second" may explicitly or implicitly include one or more features. In the descriptions of this application, "a plurality of" means two or more, unless otherwise definitely and specifically limited.

In this application, unless explicitly specified and defined otherwise, terms "install", "interconnect", "connect", "fix", and the like are to be understood in a broad sense, for example, may be a fixed connection, a detachable connection, or an integrated formation, may be a mechanical connection or an electrical connection, may be communication, may be a direct connection or an indirect connection through an intermediate medium, or may be an internal communication between two elements or an interaction relationship between two elements. A person of ordinary skill in the art may understand the specific meanings of the foregoing terms in this application according to specific situations.

In this application, unless otherwise explicitly specified or defined, the first feature being located "above" or "below" the second feature may be the first feature being in direct contact with the second feature, or the first feature being in indirect contact with the second feature through an intermediate medium. In the descriptions of this specification, descriptions of a reference term such as "an embodiment", "some embodiments", "an example", "a specific example", or "some examples" means that a feature, structure, material, or characteristic that is described with reference to the embodiment or the example is included in at least one embodiment or example of this application. In this specification, schematic expressions of the foregoing terms do not necessarily refer to the same embodiment or example. Moreover, the specific features, the structures, the materials or the characteristics that are described may be combined in proper manners in any one or more embodiments or examples.

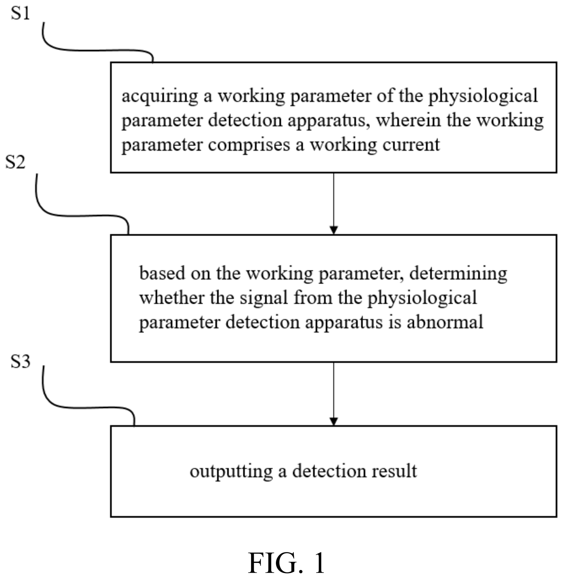

As shown in FIG. 1, this embodiment of this application provides a method of detecting an anomaly of a signal from a physiological parameter detection apparatus, comprising:

S1: acquiring a working parameter of the physiological parameter detection apparatus, wherein the working parameter comprises working current;
S2: determining whether the signal from the physiological parameter detection apparatus is abnormal based on the working parameter, and
S3: outputting a detection result.

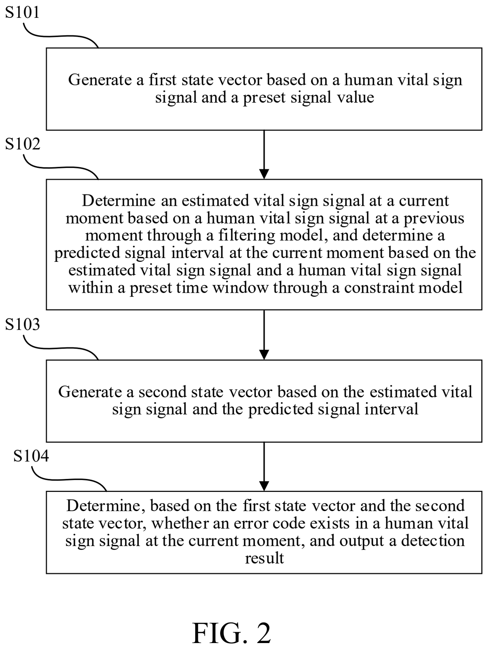

As shown in FIG. 2, in the method according to the first embodiment in embodiments of this application (particularly the method for detecting an error code in a human vital sign signal), the signal from the physiological parameter detection apparatus is a human vital sign signal, and the method further comprises:
S101: generating a first state vector based on the human vital sign signal and a preset signal value.

The human vital sign signal refers to a related signal that is acquired/collected through a corresponding physiological parameter detection apparatus or sensor, and can represent a human vital sign signal (physiological parameter signal) of a user. For example, the human vital sign signal may be a blood glucose signal of a user that is acquired by implanting a continuous glucose monitor (CGM) sensor under the skin of the user, or an electrocardiosignal of the user that is acquired by an electrocardiograph, or a blood pressure signal, a blood oxygen signal, or the like of the user that is detected through a sphygmomanometer or an oximeter. The human vital sign signal from the physiological parameter detection apparatus can be characterized by the working current of physiological parameter detection apparatus, more specifically, calculated from the working current. For example, a basic principle formula of the CGM is as follows: $GLU = \dfrac{I_w}{K_s}$, wherein GLU is a glucose concentration of a human body, that is, a glucose value; $I_w$ is a working current of the CGM; and $K_s$ is a sensitivity of the CGM.

Causes of an error code may be roughly classified into the following several types: a low current phenomenon caused by a device fault, a current saturation phenomenon caused by a short circuit between electrodes of a device, a current signal fluctuation phenomenon generated by a device or in a use process, a severe vibration phenomenon of a current signal that is generated by an abnormal electrode or in a use process, a continuously decreasing phenomenon of a current that is caused by attenuation of performance of an electrode, and the like.

A blood glucose signal is used as an example. A process of acquiring a signal through a CGM is easily affected by problems such as noise, a sensor performance drift, breakage of a screen-printed electrode, a calibration error, and wearing compression. As a result, a current obtained on a working electrode is not a Faradic current generated by an actual reaction between glucose and an enzyme. By performing error code detection (i.e. ErrorCode detection), a valid and normal working current $I_w$ is identified. This improves accuracy and reliability of monitoring blood glucose by the CGM, reduces an error rate, and facilitates analyzing and locating an error code.

Before step S101 is performed, initialization needs to be performed in advance, and a signal interval and a preset signal value that correspond to a human vital sign signal are set for the human vital sign signal. The signal interval mainly refers to intervals in which the human vital sign signal is a normal signal, intervals in which the human vital sign signal is an apparently abnormal signal, and the like. Certainly, the signal interval may be further divided into a plurality of sub-intervals based on a need, or different signal interval division manners may be set based on different scenarios. The preset signal value includes a first signal value, a second signal value, and a third signal value, and are respectively critical values of corresponding signal intervals.

Specifically, for a signal value of the human vital sign signal, a first signal interval corresponding to the signal value is generated based on a preset conventional numerical value interval of a human vital sign signal. The conventional value interval refers to an interval in which the human vital sign signal falls when a human body is normal, and may be obtained based on a corresponding medical standard or big data analysis.

A blood glucose value of a human body is used as an example. The conventional numerical value interval of a human vital sign signal is expressed by $[Glu_{min}, Glu_{max}]$, and a range of the conventional value interval of the signal value is preferably [3 mmol/L, 30 mmol/L]. This range is merely an exemplary scope in a conventional scenario. During actual execution, it may be correspondingly changed based on a change in the scenario (for example, a change based on an external condition such as the age of a user or a district of a user). Unless otherwise specified, the ranges exemplified in this embodiment of this application are all exemplary ranges, and do not represent fixed values of corresponding intervals or values. Details will not be elaborated subsequently.

The sensitivity of the physiological parameter detection apparatus (e.g. data detection sensor, such as CGM) is represented by Ks. In this case, the human blood glucose signal value GLU acquired by CGM is calculated using the

formula $GLU = \dfrac{I_w}{K_s}$ :,, where $I_w$ is the working current of the CGM; and $K_s$ is the sensitivity of the CGM. The first signal interval may be $[Glu_{min}, Glu_{max}]$, meaning a range of the working current $I_w$ is $[Glu_{min} * Ks, Glu_{max} * Ks]$.

Similarly, for a change value of a human vital sign signal, a second signal interval corresponding to the change value is generated based on the preset conventional value interval of the change value of a human vital sign signal. There are a plurality of second signal intervals which at least respectively correspond to a rising value and a falling value.

A blood glucose value of a human body is used as an example. A falling value of the blood glucose of the human body usually does not exceed 4 mmol/L to 5 mmol/L every hour. In this case, the falling value can be set to 6 mmol/L, and a conventional value interval $[Glu\_down\_drift_{min}, Glu\_down\_drift_{max}]$ of the falling value may be set to [0, 0.1 mmol/L/min]. Similarly, a rising value of the blood glucose of the human body can usually reach 0.2 mmol/L/min to 0.5 mmol/L/min. A conventional value interval $[Glu\_up\_drift_{min}, Glu\_up\_drift_{max}]$ of the rising value can be set to [0, 0.5 mmol/L/min]. In this case, for the rising value and the falling value, the conventional value intervals of the change of a human vital sign signal corresponding to the rising value and the falling value may be directly set to second signal intervals corresponding to the rising value and the falling value.

However, by using only the conventional numerical value intervals of a human vital sign signal and the conventional value intervals of the change of a human vital sign signal, it is difficult to describe the impact of other factors in a real scenario during actual measurement. Therefore, for the first signal interval, a left endpoint is corrected to a fixed value less than a current value, and a right endpoint is corrected to be a variable value that is greater than the current value and is obtained by using the current value and a first coefficient. For example, the left endpoint of the first signal interval is corrected to 0, and the right endpoint is corrected to $Glu_{max} * (1 + \alpha)$, where $\alpha$ represents a non-zero positive value, which is preferably 20%, thereby expanding the range of the right endpoint. Certainly, the right endpoint may alternatively be represented in another manner, as long as the range of the right endpoint can be expanded, thereby expanding the first signal interval and improving robustness. Correspondingly, the first signal value is set to $Glu_{max} * (1 + \alpha)$.

Similarly, for the second signal interval, the right endpoint is corrected to be a variable value that is greater than the current value and is obtained by using the current value and a second coefficient. For example, the right endpoint of the second signal interval of the falling value is corrected to $Glu\_down\_drift_{max} * (1 + \beta)$. When $Glu\_down\_drift_{max} = 0.1$ mmol/L/min, the second signal interval of the falling value is $[0, 0.1 * (1 + \beta) mmol/L/ min]$. The right endpoint of the second signal interval of the rising value is corrected to $Glu\_up\_drift_{max} * (1 + \lambda)$. When $Glu\_up\_drift_{max} = 0.5$ mmol/L/min, the second signal interval of the rising value is $[0, 0.5 * (1 + \lambda) mmol/L/min]$. Where both $\beta$ and $\lambda$ are non-zero positive values and are preferably 30%, thereby expanding the range of the right endpoint. Certainly, the right endpoint may alternatively be represented in another manner, as long as the range of the right endpoint can be expanded, thereby expanding the second signal interval and improving robustness. Correspondingly, the second signal value is set to $Glu\_up\_drift_{max} * (1 + \lambda)$, and the third signal value is set to $Glu\_down\_drift_{max} * (1 + \beta)$.

A corresponding human vital sign signal is obtained after initialization. In this case, an initialized signal interval and an initialized preset signal value are determined for the human vital sign signal. For different human vital sign signals, different signal intervals and preset signal values may be respectively initialized.

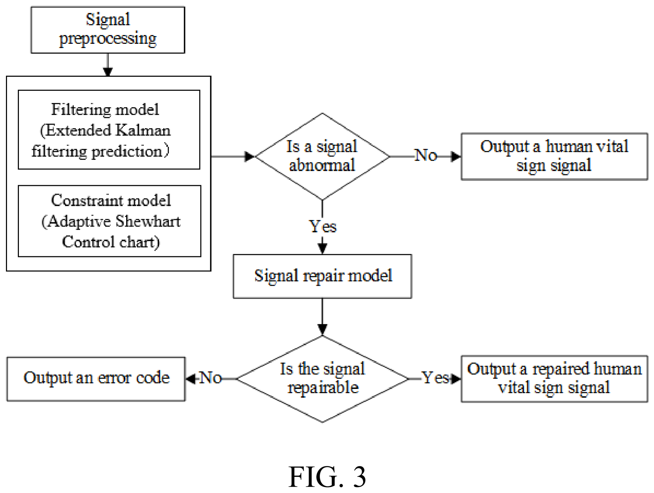

As shown in FIG. 3, the human vital sign signal is preprocessed based on the preset signal value, so as to update the human vital sign signal. A main objective of preprocessing is to update a human vital sign signal with a corresponding value exceeding the preset signal value, to facilitate subsequent processing. Certainly, a signal that has been preprocessed may be correspondingly marked, so as to determine, in a subsequent processing process based on the mark, whether the signal has been updated.

Specifically, after acquiring the working parameter of the physiological parameter detection apparatus, particularly the working current of the apparatus, an inputted human vital sign signal $CgmSignal_i$ is obtained, here taking the continuous glucose monitor (CGM) as an example, where i represents an $i^{th}$ moment. If a signal value of the human vital sign signal exceeds the first signal value, namely $CgmSignal_i > Glu_{max} * (1 + \alpha)$, the first signal value $Glu_{max} * (1 + \alpha)$ is used as an update value of the human vital sign signal.

Based on the human vital sign signal, a change value $\Delta Signal_i$ of the human vital sign signal is obtained by using $\Delta Signal_i = CgmSignal_i - CgmSignal_{i-1}$, and $CgmSignal_{i-1}$ represents a human vital sign signal at an $(i-1)^{th}$ moment, where the change value includes a rising value and a falling value. For the rising value, if the change value of the human vital sign signal exceeds the second signal value corresponding to the rising value, namely $\Delta Signal_i > Glu\_up\_drift_{max} * (1 + \lambda)$, the human vital sign signal $CgmSignal_i$ is updated to $CgmSignal_{i-1} + Glu\_up\_drift_{max} * (1 + \lambda)$. Certainly, this value cannot exceed the first signal value $Glu_{max} * (1 + \alpha)$.

Similarly, for the falling value, if the change value of the human vital sign signal exceeds the third signal value corresponding to the falling value, namely $\Delta Signal_i < < -Glu\_down\_drift_{max} * (1 + \beta)$, the human vital sign signal $CgmSignal_i$ is updated to $CgmSignal_{i-1} - Glu\_down\_drift_{max} * (1 + \beta)$. Certainly, this value needs to be greater than 0.

After the human vital sign signal is preprocessed to obtain a vital sign update signal, the vital sign update signal meets a requirement of the preset signal value. In this case, the first state vector is generated based on a preprocessed human vital sign signal and the preset signal value.

The vital sign update signal is represented in a vector form, thus obtaining a signal vector. The first state vector mainly indicates whether the signal vector is updated because the signal vector exceeds the preset signal value.

Specifically, when the vital sign update signal $CgmSignal_i \geq Glu_{max} * (1 + \alpha)$, or $CgmSignal_i = 0$, or $\Delta Signal_i \geq Glu\_up\_drift_{max} * (1 + \lambda)$, or $\Delta Signal_i \leq -Glu\_down\_drift_{max} * (1 + \beta)$, it indicates that the vital sign update signal is updated because the signal vector exceeds the preset signal value. In this case, a corresponding first state vector $CgmStatus_i$ is recorded as a first preset value of 0, and may be recorded as a second preset value of 1 in other cases.

Two groups of vector data are obtained after the human vital sign signal is processed as above:

a set $Pro\_CgmSignal_n = [Pro\_CgmSignal_1, Pro\_CgmSignal_2 ... Pro\_CgmSignal_n]$ corresponding to the human vital sign signal, where $Pro\_CgmSignal_i$ refers to a vital sign update signal which was preprocessed and updated, and n represents a total number of moments; and

a set $CgmStatus_n = [CgmStatus_1, CgmStatus_2 ... CgmStatus_n]$ corresponding to the first state vector.

In this case, the signal vector and the first state vector are used as inputs of a filtering model and a constraint model, to facilitate subsequent identification of an error code.

S102: Determine an estimated vital sign signal at a current moment based on a human vital sign signal at a previous moment through a filtering model; and determine a predicted signal interval at the current moment based on a human vital sign signal within a preset time window through a constraint model.

A blood glucose value is used as an example, use or interference exists in an actual CGM signal system. Consequently, an acquired blood glucose value signal is nonlinear. In a prediction process of the filtering model, a nonlinear system may be approximately regarded as a linear system, thereby obtaining an accurate prediction result.

Based on this, as shown in FIG. 3, in the nonlinear system, the filtering model performs Taylor series expansion around a filtering value on a state equation and an observation equation that are nonlinearized, and second-order and higher-order terms are deleted, thereby linearizing the state equation. The nonlinear system is approximated as a linear system, and then a standard Kalman filtering algorithm is used to estimate the signal vector of the human vital sign signal.

For elaboration, a standard Kalman filtering technology is usually applicable to making optimal estimation on a target state under a condition of a linear Gaussian model. Its state equation is: $x_{k+1} = f(x_k) + W_k$, and its observation equation is: $y_k = h(x_k) + v_k$, where the state equation f and the observation equation h are nonlinear functions; k represents discrete time; process noise $W_{k-1}$ and observation noise $v_k$ are respectively irrelevant and satisfy a Gaussian distribution with covariances of $Q_k$ and $R_k$.

It is assumed that a state estimate $\overline{x}_{k|k}$ and an estimated variance $P_{k|k}$ at a k moment are known, and Taylor series expansion is performed on a state equation and an observation equation. After higher-order items are ignored, the state equation is $x_{k+1} \approx f(\overline{x}_{k|k}) + F_k(x_k - \overline{x}_{k|k})$ and the observation equation is $y_{k+1} \approx h(\overline{x}_{k+1|k}) + H_{k+1}(x_{k+1} - \overline{x}_{k+1|k})$. Where $F_k$ and $H_k$ are respectively jacobian matrixes of the state equation f and the observation equation h at $\overline{x}_k$.

In this case, a linearized filtering model is used for prediction. The filtering model includes: $\overline{x}_{k+1|k} = f(\overline{x}_{k|k})$ and $P_k$

$_{+1|k} = F_k P_k F_k{}^T + Q_k$. Where $\bar{x}_{k+1|k}$ represents prediction on an estimated feature signal $x_{k+1}$ at a $(k+1)^{th}$ moment based on information at the k moment; $P_{k+1|k}$ represents a predicted covariance matrix; and $Q_k$ represents a covariance matrix of the process noise.

It should be noted that when a new inputted signal $y_{k+1|k}$ is determined as a valid signal, the state estimate needs to be updated to obtain an optimal estimation result corresponding to the inputted signal for application to signal detection at a next moment. A determining process of the corresponding optimal estimation result is as follows:

$$K_{k+1} = P_{k+1|k} H_{k+1}{}^T (H_{k+1} P_{k+1|k} H_{k+1}{}^T + R_{k+1})^{-1};$$

$$\bar{x}_{k+1|k+1} = \bar{x}_{k+1|k} + K_{k+1}(y_{k+1} - h(\bar{x}_{k+1|k}));$$

$$P_{k+1|k+1} = (I - K_{k+1} H_{k+1}) P_{k+1|k},$$

where $K_{k+1}$ represents a Kalman gain; $\bar{x}_{k+1|k+1}$ represents an updated state estimate; $P_{k+1|k+}$ represents an updated covariance matrix; and $R_{k+1}$ represents a covariance matrix of the observation noise.

Based on this, when prediction is performed through the filtering model, the state equation f and the observation equation h that are included in the filtering model are first determined, and initial values of parameters of the filtering model are set. Setting initial values of parameters required in an estimation process mainly includes distribution of initial states, an initial distribution of a covariance matrix of the distribution of the initial states, a process noise covariance matrix, and a measurement noise covariance matrix, and the like.

By using the state equation, a first predicted value of a human vital sign signal at a current moment (the $(k+1)^{th}$ moment) is obtained based on an optimal estimation result of a human vital sign signal at a previous moment (the $k^{th}$ moment).

A linearized state equation is obtained by calculating a jacobian matrix of a nonlinear state equation, and a second predicted value of an error covariance matrix at the current moment (the $(k+1)^{th}$ moment) based on the linearized state equation and an error covariance matrix at the previous moment (the $k^{th}$ moment).

A filter gain is obtained based on the first predicted value and the second predicted value. The filter gain is used for measuring a weight of old and new information. An optimal estimation result of the human vital sign signal at the current moment (the $(k+1)^{th}$ moment) and a posteriori estimate of the error covariance matrix are obtained based on the filter gain and a measured value of a human vital sign signal at the previous moment (the $k^{th}$ moment). Finally, the process is repeated until a final moment is calculated.

Certainly, in addition to the standard Kalman filtering algorithm, another filtering algorithm may be used, for example, an extended Kalman filtering algorithm, an unscented Kalman filtering algorithm, or a particle filtering algorithm. In some filtering algorithms, vector estimation may be performed without linearizing the state equation, thereby improving estimation accuracy and increasing algorithm complexity. Selection may be performed based on an actual case.

Certainly, in addition to the filtering algorithm, for different scenarios, a time series analysis algorithm such as an autoregressive moving average model or an autoregressive integrated moving average model, or a neural network algorithm such as a recursive neural network or a long short-term memory network, may be used to estimate a signal vector based on an actual need.

The filtering model is mainly used for estimating the human vital sign signal, and the constraint model is mainly used for constraining the predicted signal interval, so as to facilitate determining, in a subsequent signal processing process, whether an error code exists in the inputted signal.

Specifically, as shown in FIG. 3, constructing a constraint model by using a Shewhart control chart is used as an example for explanation.

First, an appropriate time window value N is selected. For a human vital sign signal within a preset time window N, a mean and a standard deviation that correspond to the human vital sign signal are determined.

An mean $Ave\_Pro\_CgmSignal_N$ corresponding to [$Pro\_CgmSignal_{i-N-1}$, $Pro\_CgmSignal_{i-1}$] time window N is calculated based on $Ave\_Pro\_CgmSignal_N = \frac{1}{N}\sum_{i=1}^{N}(Pro\_CgmSignal_i)$.

A standard deviation $Std\_Pro\_CgmSignal_N$ corresponding to $Pro\_CgmSignal_{i-N-1}$, $Pro\_CgmSignal_{i-1}$] time window N is calculated based on

$$Std\_Pro\_CgmSignal_N = \sqrt[2]{\frac{\sum_{i=1}^{N}(Pro\_CgmSignal_i - Ave\_Pro\_CgmSignal_N)^2}{N}}.$$

At least one of the mean and the standard deviation and a human vital sign signal at the previous moment are inputted to the constraint model based on the Shewhart control chart, and a maximum critical value and a minimum critical value of the predicted signal interval of the human vital sign signal at the current moment are outputted.

Specifically, positive compensation is performed on the human vital sign signal at the previous moment by using a third preset coefficient and the standard deviation, to obtain the maximum critical value of the predicted signal interval at the current moment. In addition, negative compensation is performed on the human vital sign signal at the previous moment by using a fourth preset coefficient and the standard deviation, to obtain the minimum critical value of the predicted signal interval at the current moment. Values of the third preset coefficient and the fourth preset coefficient are positively correlated with the human vital sign signal at the previous moment.

For example, in conjunction with information of the human vital sign signal $Pro\_CgmSignal_{i-1}$ at the previous moment and the estimated vital sign signal $\bar{x}_{k+1|k}$ at the current moment, the mean $Ave\_Pro\_CgmSignal_N$ and the standard deviation $St\_Pro\_CgmSignal_N$ are inputted to a customized Shewhart control chart constraint model to obtain the predicted signal interval of the human vital sign signal $CgmSignal_i$ at the current moment.

The customized Shewhart control chart constraint model may be:

$$\text{upLimit\_CgmSignal}_i, \text{lowLimit\_CgmSignal}_i$$
$$= f\big(\text{Ave\_Pro\_CgmSignal}_N, \bar{x}_{k+1|k}, \text{Std\_Pro\_CgmSignal}_N, \text{Pro\_CgmSignal}_{i-1}\big)$$

where the maximum critical value is:

$$\text{upLimit\_CgmSignal}_i = G(\text{Pro\_CgmSignal}_{i-1}, \text{Ave\_Pro\_CgmSignal}_N, \bar{x}_{k+1|k}) + \gamma *$$
$$\text{Std\_Pro\_CgmSignal}_N,$$

the minimum critical value is:

$$\text{LowLimit\_CgmSignal}_i = G(\text{Pro\_CgmSignal}_{i-1}, \text{Ave\_Pro\_CgmSignal}_N, \bar{x}_{k+1|k}) - \beta *$$
$$\text{Std\_Pro\_CgmSignal}_N,$$

where $\gamma$ and $\beta$ respectively represent the third preset coefficient and the fourth preset coefficient, coefficient values of which need to be determined based on $Pro\_CgmSignal_{i-1}$. When $Pro\_CgmSignal_{i-1}$ is relatively small, the coefficient values of $\gamma$ and $\beta$ are decreased, and when $Pro\_CgmSignal_{i-1}$ is relatively large, the coefficient values of $\gamma$ and $\beta$ are increased, thus implementing a positive correlation. The $\gamma$ and $\beta$ coefficients are non-zero positive values. Values of the third preset coefficient and the fourth preset coefficient are positively correlated to the value of the human vital sign signal at the previous moment and can implement corresponding positive compensation and negative compensation, and are not limited to the manners in the above formulas.

Certainly, in addition to a Shewhart control chart, a similar function may be implemented by using a manner such as a cusum control chart, a control chart of exponential weighted moving average, or a single value-moving R-control chart, to determine an upper track and a lower track of the signal interval.

Or, the maximum critical value and the minimum critical value of the signal interval may be determined in a manner such as an autoregressive model, a neural network, or support vector regression based on a need.

S103: Generate a second state vector based on the estimated vital sign signal and the predicted signal interval.

Correspondingly, if the estimated vital sign signal falls within the predicted signal interval, a state value corresponding to the estimated vital sign signal is determined as a second preset value; if the estimated vital sign signal does not fall within the predicted signal interval, the state value corresponding to the estimated vital sign signal is determined as a first preset value; and the second state vector is generated based on the state value of each estimated vital sign signal.

Specifically, a priori estimate value $\bar{x}_{k/k-1}$ at the current moment is calculated based on the filtering model by using an optimal state estimate value $\bar{x}_{k-1}$ at the previous moment and a measurement signal $x_k$ at the current moment, and whether $\bar{x}_{k|k-1}$ falls within the predicted signal interval [$LowLimit\_CgmSignal_i$, $upLimit\_CgmSignal_i$] is determined; if yes, a state value $CgmInGap_i$ of the second state vector is recorded as the second preset value of 1; and if no, the state value is recorded as the first preset value of 0. Thus, the second state vector [$CgmInGap_1$, $CgmInGap_2$, $CgmInGap_3$ ...] is generated.

S104: Determine, based on the first state vector and the second state vector, whether an error code exists in a human vital sign signal at the current moment, and output the detection result.

As shown in FIG. 3, if no abnormal signal exists, the human vital sign signal is directly outputted, and if an abnormal signal exists, whether the abnormal signal is repairable is further determined. The abnormal signal may be determined based on whether the estimated signal vector falls within an estimated signal interval. If the estimated signal vector does not fall within the estimated signal interval, the abnormal signal exists.

Specifically, for a human vital sign signal at a single moment, if a first state vector corresponding to the human vital sign signal represents that a change state during preprocessing does not change (that is, the first state vector is recorded as 1 in the set corresponding to the first state vector), and a second state vector corresponding to the human vital sign signal represents that the second state vector falls within the estimated signal interval (that is, the second state vector is recorded as 1 in the set corresponding to the second state vector), the human vital sign signal at the single moment is used as a valid signal, and a valid sequence is recorded as $CgmValid_n = CgmStatus_n \cap CgmInGap_n$.

$$CgmValid_i = \begin{cases} 1, CgmInGap_i = 1 \ and \ CgmStatus_i = 1 \\ 0, otherwise \end{cases},$$

where $CgmStatus_i$ represents the first state vector, and $CgmInGap_i$ represents the second state vector.

Based on this, a vector $XorCgmVaild = CgmValid \oplus M$ is set, where a vector $M = [1,1,1 ... ]$, and a vector $CgmValid$ is a vector composed of a valid signal $CgmValid_i$.

If a count of continuous 1 in the vector $XorCgmVaild$ is not less than a preset threshold Err_Thd, it indicates that the signal is continuously preprocessed and updated or does not fall within the estimated signal interval, unrepairable error code information is outputted. Otherwise, it is determined that the error code is repairable, and no error code information is outputted. A code repairing process may be similar to the signal preprocessing process described above, that is, an excessively large or small signal value is repaired to be within a proper interval.

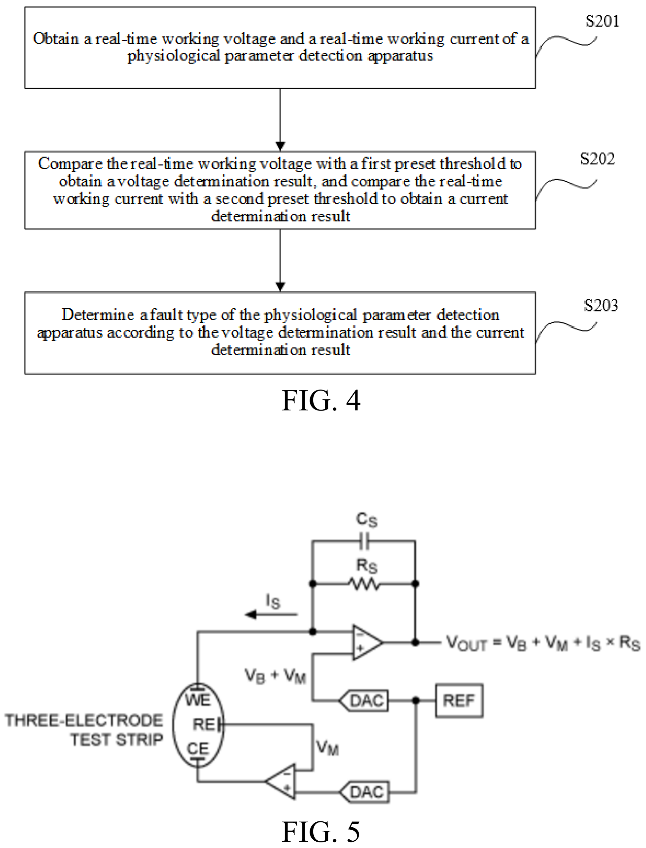

Fig. 4 is a schematic flowchart of a second embodiment in embodiments of this application (particularly a method for detecting a fault type of a physiological parameter detection apparatus). The working parameter of the physiological parameter detection apparatus comprises the working voltage and the working current, wherein the working voltage comprises a real-time working voltage and the working current comprises a real-time working current. As show in Fig. 4, the method further comprises:

S201: Obtain a real-time working voltage and a real-time working current of a physiological parameter detection apparatus.

The physiological parameter detection apparatus generally collects (acquires) a physiological signal of a human body (a human vital sign signal) based on a sensor. Using a Continuous Glucose Monitor (CGM) shown in FIG. 5 as an example, when a sensor micro-needle is implanted into a body, an enzyme layer of a working electrode undergoes an electrochemical reaction with glucose in tissue liquid to generate a weak current (level nA). A circuit is formed between the working electrode and a counter electrode. Then, a current of the circuit is measured and a concentration of the glucose is calculated by using an algorithm. A stable voltage is provided for the working electrode for continuous and stable response, to ensure a stable oxidation reaction of the glucose and enzyme on the working electrode. The CGM fluctuates randomly when having a fault $I_w$. In this case, a blood glucose value measured $I_w$ by the working electrode is not a real blood glucose value of the human body. Therefore, to quickly capture a change of a working signal of the apparatus, a timely information source is provided for fault detection, so that a fault can be detected in an early stage, and an invalid signal can be prevented from being continuously used. In the second embodiment of this application, the real-time working voltage and the real-time working current of the physiological parameter detection apparatus are obtained.

As shown in FIG. 5, in an embodiment, the real-time working voltage includes a working electrode voltage $WEVol_i$, a reference electrode voltage $REVol_i$, and a counter electrode voltage $CEVol_i$. By monitoring a real-time working voltage and a real-time working current of a physiological parameter detection apparatus in real time, a change of a working signal of the apparatus can be quickly captured, and a timely information source is provided for fault detection, so that a fault can be detected in an early stage, and continuity and stability of a device can be ensured for a physiological parameter detection apparatus that needs to continuously operate, thereby improving reliability and accuracy of the physiological parameter detection apparatus.

In an embodiment, to ensure stable performance and an accurate measurement capability of a sensor electrode at an initialization stage, so as to ensure that a reliable real-time working current and real-time working voltage are obtained, before obtaining the real-time working voltage and the real-time working current of the physiological parameter detection apparatus, the method further includes the following process.

First, an initial voltage of the physiological parameter detection apparatus is collected according to a preset sampling period. For example, the preset sampling period is 0.5 hour, and if a sampling rate is 3 min/Point, initial voltages of 10 points may be obtained. In this case, an average voltage, a standard deviation and a range value that correspond to the initial voltage may be calculated, thereby determining whether the average voltage, the standard deviation, and the range value are all within corresponding preset thresholds. If the average voltage, the standard deviation and the range value are all within corresponding preset thresholds, it indicates that the physiological parameter detection apparatus has a reliable measurement capability. In this case, the real-time working voltage and the real-time working current of the physiological parameter detection apparatus can be obtained.

Specifically, first, an initial voltage of the physiological parameter detection apparatus needs to be obtained after a sensor of the physiological parameter detection apparatus completes a polarization process, so as to calculate, based on the following formula, the average voltage, the standard deviation, and the range value that correspond to the initial voltage. When the initial voltage includes the working electrode voltage and the reference electrode voltage, an average voltage of the working electrode voltage is: $WEAvr = \frac{1}{n}\sum_{i=1}^{n} \text{WEVol}_i$, where *WEAvr* is an average voltage of the working electrode voltage, n is a number of the working electrode voltages collected in the preset sampling period, i=1, 2, ..., n, and represents a current $i^{th}$ voltage, and WEVol$_i$ represents an $i^{th}$ working electrode voltage collected in the sampling period.

A standard deviation of the working electrode voltage is: $WEStd = \sqrt{\frac{1}{n}\sum_{i=1}^{n}(\text{WEVol}_i - WEAvr)^2}$, where *WEStd* is the standard deviation of the working electrode voltage.

A range value of the working electrode voltage is: $WEMax_{diff}$ = Max(WEVol$_i$)-Min(WEVol$_i$), where $WEMax_{diff}$ is the range value of the working electrode voltage, and Max(WEVol$_i$) is a maximum value of the working electrode voltage collected in the preset sampling period, and Min(WEVol$_i$) is a minimum value of the working electrode voltage collected in the preset sampling period.

An average voltage of the reference electrode voltage is: $REAvr = \frac{1}{n}\sum_{i=1}^{n} \text{REVol}_i$, where *REAvr* is the average voltage of the reference electrode voltage, i=1, 2,..., n, and represents a current $i^{th}$ voltage, and REVol$_i$ represents an $i^{th}$ reference electrode voltage collected in the sampling period.

A standard deviation of the reference electrode voltage is: $REStd = \sqrt{\frac{1}{n}\sum_{i=1}^{n}(\text{REVol}_i - REAvr)^2}$, where *REStd* is the standard deviation of the reference electrode voltage.

A range value of the reference electrode voltage is: $REMax_{diff}$ = Max(REVol$_i$)-Min(REVol$_i$), where $REMax_{diff}$ is the range value of the working electrode voltage, Max(REVol$_i$) is a maximum value of the reference electrode voltage collected in a preset sampling period, and Min(REVol$_i$) is a minimum value of the reference electrode voltage collected in the preset sampling period.

Whether the average voltage, the standard deviation, and the range value are all within corresponding preset thresholds may be determined based on the following conditions:

Condition 1: *WEAvr* ∈ [*WESetMinVoltage, WESetMaxVoltage* ], that is, whether the average voltage of the working electrode falls within a threshold of an acceptable range of the working electrode; The working electrode voltage, for example, can be set to the electrochemical operating voltage specified by the manufacturer for different sensor characteristics, typically a fixed value V_set. Due to variations in the internal reference voltage of the chip and fluctuations in the DAC, the maximum and minimum deviations are generally within ±10mV;

Condition 2: *WEStd* ∈ *[WESetMinStd, WESetMaxStd* ] , that is, whether the standard deviation of the working electrode voltage falls within a threshold of an acceptable range of the standard deviation of the working electrode voltage, and how to determine the acceptable range of the standard deviation of the working electrode voltage; As mentioned above, the WE (working electrode) voltage is typically a fixed value, and the acceptable standard deviation is generally 5 mV (i.e., slightly greater than the maximum deviation/2.1);

Condition 3: $WEMax_{diff}$ < $WESetMax_{diff}$, that is, whether a maximum difference in the measurement of the working electrode voltage is less than a set upper limit value of the working electrode voltage;

Condition 4: *REAvr* ∈ [*RESetMinVoltage, RESetMaxVoltage* ], that is, whether the average voltage of the reference electrode is within the threshold of the acceptable range of the average voltage of the reference electrode; The voltage of the reference electrode (RE), for example, can be set as the electrochemical operating voltage configured by the manufacturer for different sensor characteristics, typically a fixed value V_set. Due to variations in the internal reference voltage of the chip and fluctuations in the DAC, the maximum and minimum deviations are generally within ±10mV;

Condition 5: *REStd* ∈ *[RESetMinStd, RESetMaxStd* ], that is, whether the standard deviation of the reference electrode voltage is within a threshold of an acceptable range of the standard deviation of the reference electrode voltage; As mentioned above, the RE (reference electrode) voltage is typically a fixed value, and the acceptable standard deviation is generally 5 mV (i.e., slightly greater than the maximum deviation/2.1);

Condition 6: $REMax_{diff} < RESetMax_{diff}$, that is, whether the maximum difference in measurement of the reference electrode voltage is within an acceptable upper limit of the maximum difference of the reference electrode voltage; and

Condition 7: WorkVolSet = $WEAvr$ - $REAvr$,

WorkVolSet $\in$ [WorkVolSet*MinVoltage,* WorkVolSet*MaxVoltage* ] , that is, the working voltage is set within an acceptable range of a working voltage setting, and the working voltage setting is usually calculated by using a difference between an average voltage of the working electrode (WEAvr) and an average voltage of the reference electrode (REAvr). As mentioned above, the working electrode (WE) and reference electrode (RE) can generally be set to a stable voltage during normal electrochemical reactions, so WorkVolSet can be a fixed value. Due to variations in the internal reference voltage of the chip and DAC fluctuations, the maximum and minimum deviations are typically within $\pm10mV$.

If it is determined that the average voltage, the standard deviation, and the range value all fall within the preset thresholds corresponding to the foregoing Condition 1 to Condition 7, it indicates that the physiological parameter detection apparatus has a reliable measurement capability. In this case, the real-time working voltage and the real-time working current of the physiological parameter detection apparatus can be obtained.

S202: Compare the real-time working voltage with a first preset threshold to obtain a voltage determination result, and compare the real-time working current with a second preset threshold to obtain a current determination result.

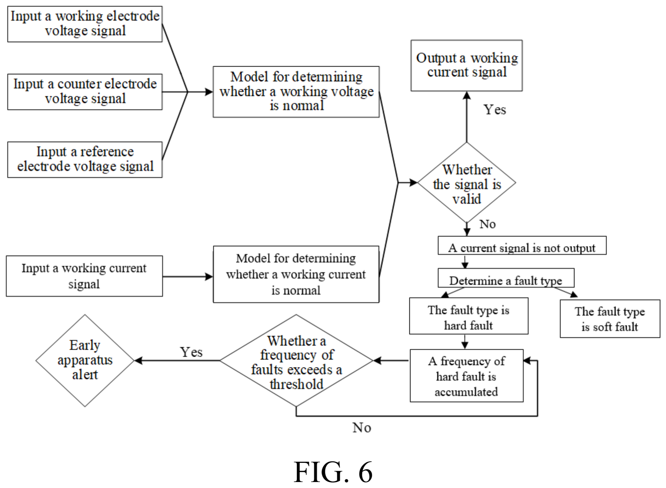

A current physiological parameter detection apparatus is prone to be affected by problems such as noise, sensor performance drift, breakage of a screen-printed electrode, wearing compression, and bleeding short circuit when continuously detecting a physiological parameter, causing a fault of the physiological parameter detection apparatus. Therefore, to facilitate determining whether an abnormality occurs on the physiological parameter detection apparatus, in the second embodiment of this application, as shown in FIG. 6, a real-time working voltage is compared with a first preset threshold to obtain a voltage determination result, and a real-time working current is compared with a second preset threshold to obtain a current determination result. By performing dual monitoring on both a voltage and a current, a working state of the physiological parameter detection apparatus can be understood more comprehensively. In addition, for a wearable physiological parameter detection apparatus, abnormalities of the voltage and the current are found in time, so that an invalid signal collected by a faulty apparatus can be prevented from being continuously used, thereby helping to improve reliability of the physiological parameter detection apparatus.

Specifically, in an embodiment, based on a working circuit shown in FIG. 5 and FIG. 6, the real-time working voltage includes a working electrode voltage $WEVol_i$, a reference electrode voltage $REVol_i$, and a counter electrode voltage $CEVol_i$. The first preset threshold includes a first preset voltage threshold, a second preset voltage threshold, and a preset dropout threshold. In this case, the comparing the real-time working voltage with the first preset threshold to determine a voltage determination result includes the following process:

The tested real-time working voltage is input to the following model for determining whether a working voltage is normal:

$$VolLoopResult_i = func\_ProcessVol(\text{WEVol}_i, \text{REVol}_i, \text{CEVol}_i)$$

where $VolLoopResult_i$ is a voltage determination result.

According to the foregoing model, first, the working electrode voltage is compared with the first preset voltage threshold, and the reference electrode voltage is compared with the second preset voltage threshold, to determine a first voltage determination result. That is, in a voltage determining process, whether a voltage signal is stable is first determined by using a determination model. That is, whether the voltage values input by the working electrode and the reference electrode are stable values needs to be determined. When fluctuations generated by the input voltage signal WEVol*i* and $RE$Vol$_i$ exceed set thresholds, it indicates that the first voltage determination result is an abnormality. In addition, because there is a stable voltage difference in an electrochemical reaction process of the physiological parameter detection apparatus, in a process of detecting an abnormality, voltage differences between the working electrode voltage and the counter electrode voltage and between the reference electrode voltage and the counter electrode voltage further need to be determined, to determine a second voltage determination result according to the voltage difference and a corresponding preset voltage difference threshold. If there is an abnormal result in the first voltage determination result or the second voltage determination result, it indicates that the current voltage determination result $VolLoopResult_i$ indicates an abnormality, error processing needs to be performed.

The working electrode voltage, the reference electrode voltage, and the counter electrode voltage are simultaneously monitored, and are compared with the preset voltage threshold. This process can comprehensively detect a voltage abnormal case in an electrochemical system. Such comprehensive detection helps to find and process a potential problem in time, thereby improving stability and reliability of the physiological parameter detection apparatus. Stability of the voltage signal, that is, whether the voltage value fluctuates within a stable range, is determined. This process can ensure that a voltage in the electrochemical system is in a stable state. A voltage difference affects a rate and a direction of the electrochemical reaction in the physiological parameter detection apparatus. Therefore, whether a current electrochemical reaction of the physiological parameter detection apparatus operates normally can be determined in time by detecting a voltage difference between the working electrode voltage and the counter electrode voltage and between the reference electrode voltage and the counter electrode voltage and comparing the voltage difference with a preset voltage difference threshold, that is, finding out a voltage abnormality in time, thereby helping to reduce overall maintenance costs of the physiological parameter detection apparatus.

In an embodiment, referring to FIG. 5, the preset voltage difference threshold includes a first preset voltage difference threshold and a second preset voltage difference threshold, and the determining a second voltage determination result includes the following process:

a working electrode end WE and a counter electrode end CE are a circuit through which an electrochemical reaction generates a current. According to a principle of a virtual short circuit of an operational amplifier, a working electrode voltage of the working electrode end is equal to a voltage set by a Digital-to-Analog Converter (DAC). There is a stable voltage difference *Vol_WR* between the working electrode voltage and the reference electrode voltage, and the voltage of the reference electrode end RE is equal to the voltage set by the DAC according to the principle of the virtual short circuit of the operational amplifier, so *Vol_WR* is a stable value. Therefore, whether the first voltage difference between the working electrode voltage and the counter electrode voltage exceeds the first preset voltage difference threshold is determined by using a model for determining whether a working voltage is normal. That is, the first voltage difference is compared with the first preset voltage difference threshold to obtain a first comparison result. In addition, because the counter electrode end is connected to an output end of the operational amplifier, and a voltage is not fixed, but the voltage of the counter electrode end needs to be lower than the voltage of the reference electrode end and the voltage of the working electrode end, a second voltage difference between the reference electrode voltage and the counter electrode voltage needs to be within the second preset voltage difference threshold. Therefore, the second voltage difference needs to be compared with the second preset voltage difference threshold to obtain the second comparison result. A second voltage determination result *VolLoopResult$_i$* is determined as an abnormality if there is a result that exceeds a corresponding preset voltage difference threshold between the first comparison result and the second comparison result.

In the process, once the voltage difference exceeds the preset voltage difference threshold, an alerting signal can be sent immediately in this process, and the second voltage determination result is determined as an abnormality. Such a rapid early fault alert and response mechanism not only improves fault detection efficiency, but also helps to avoid a problem that incorrect physiological parameter detection data is used due to output of an invalid signal, thereby improving reliability of the physiological parameter detection apparatus.

In an embodiment, referring to FIG. 6, the comparing a real-time working current with a second preset threshold to determine a current determination result includes the following process:

The tested real-time working voltage is input to the following model for determining whether a working current is normal:

$$CurrLoopResult_i = func\_ProcessCurr(InCurr_i)$$

where *CurrLoopResult$_i$* is a current determination result, and *InCurr$_i$* is a real-time working current.

Because the physiological parameter detection apparatus is affected by circuit noise, contact, drift, compression, and the like, a current value corresponding to the real-time working current sometimes is not a current value that can reflect a physiological parameter detection value. Therefore, in this embodiment, to determine whether the real-time working current is affected by an interference factor to cause an abnormal case, a first scope value corresponding to detection sensitivity of the physiological parameter detection apparatus is obtained, and a second scope value corresponding to the to-be-detected physiological parameter is obtained. Then, a threshold interval of the real-time working current is determined according to the first scope value and the second scope value. The determination model determines the real-time working current based on the threshold interval, to obtain a first current determination result.

For example, in a physiological parameter detection apparatus such as a CGM, in a normal case, a working current of a sensor of the CGM and a glucose concentration of a human body have a linear directly proportional mapping relationship, and a conversion coefficient between the working current and the glucose concentration is defined as in-vivo sensitivity. This is specifically expressed as: $GLU = \frac{I_w}{K_s} = \frac{InCurr_i}{K_s}$, where *GLU* is the glucose concentration of the human body, $I_w$ is the working current of the CGM sensor, and $K_s$ is the in-vivo sensitivity. It can be learned based on the expression that the real-time working current is *InCurr$_i$* = *GLU* * $K_s$. Because Ks is a sensitivity value of a glucose sensor, in a standardized production condition, there is a first scope value within a normal range, and in a human body system, GLU has a second scope value within a normal scope value. Therefore, it may be determined that a normal real-time working current *InCurr$_i$* has a determined scope value *[MinInCurr, MaxIncurr]* according to the first scope value and the second scope value. The determination model determines whether the input real-time working current *InCurr$_i$* exceeds a set threshold interval, so that the first current determination result may be obtained.

A fluctuation of a blood glucose value of the human body is a low-frequency signal. Therefore, signal filtering processing is performed on the real-time working current, and a second current determination result is determined according to a processed real-time working current and a preset current threshold, so that a case in which the real-time working current exceeds the threshold caused by cases such as high-frequency vibration and sudden rising and sudden falling can be determined. In addition, for cases such as performance attenuation or damage to an outer membrane that occur on the sensor, signal drift occurs in the real-time working current in a relatively long time. Therefore, low-pass filtering needs to be performed on the real-time working current, to obtain a baseline of the real-time working current. A third current determination result may be determined according to drift data of the baseline and a corresponding preset drift data threshold. It may be understood that when the baseline drifts beyond a normal fluctuation within a long-time range, the third current determination result indicates an abnormality, and error processing needs to be performed. If there is an abnormal result in the foregoing first current determination result, second current determination result, or third current determination result, a current determination result $CurrLoopResult_i$ of a real-time working current indicates an abnormality, that is, an output of a model for determining whether a working current is normal is an abnormality.

Optionally, the step of comparing a real-time working current with a second preset threshold to determine a current determination result in the second embodiment may also be combined with or replaced by the step of determining whether the human vital sign signal is abnormal in the first embodiment. Specifically, the model for determining whether a working current is normal in the second embodiment may also be combined with or replaced by the steps in the first embodiment for preprocessing, filtering, and constraining the working current corresponding to the human vital sign signal.

Further, in an embodiment, the method further includes: it is determined that the real-time working current is an invalid signal if the voltage determination result or the current determination result is an abnormality. As shown in FIG. 6, when the voltage determination result output by a model for determining whether a working system is normal is an abnormality or the current determination result output by the model for determining whether a working current is normal is an abnormality, a real-time working current signal is an invalid signal as the detection result. When an abnormal case occurs, the real-time working current that can reflect the physiological parameter detection data is determined as an invalid signal, so that the use of abnormal detection outputs can be avoided, thereby ensuring reliability of the physiological parameter detection apparatus, and avoiding further use of an erroneous result.

Preferably, in an embodiment, the method further comprises:

S203: Determine a fault type of the physiological parameter detection apparatus according to the voltage determination result and the current determination result.

Faults existing in the current physiological parameter detection apparatuses may be classified into two types: hard faults and soft faults. The hard faults are mainly because a problem occurs in the sensor or the physiological parameter detection apparatus, and in this case, the working current signal is an invalid signal. The soft faults are mainly generated in a use process of the physiological parameter detection apparatus, and are mostly correctable faults. As shown in FIG. 6, whether the real-time working current is valid can be determined after the voltage determination result and the current determination result are obtained. In this case, a specific type of a fault can be further determined by combining the voltage determination result and the current determination result, thereby facilitating targeted maintenance for the fault corresponding to the physiological parameter detection apparatus.

Specifically, in an embodiment, the fault type includes a soft fault and a hard fault, and the determining a fault type of the physiological parameter detection apparatus according to the voltage determination result and the current determination result includes the following process: if the voltage determination result is an abnormality, that is, the voltage determination result $VolLoopResult_i$ is an abnormality, it may be determined that the fault type is the hard fault. It may be learned based on the foregoing content that the voltage determination result $VolLoopResult_i$ is a result obtained after the real-time working voltage is processed by a function $func\_ProcessVol$ in the foregoing process. If the third current determination result is an abnormality, that is, a drift case occurs, the fault type may alternatively be determined as the hard fault. It may be learned based on the foregoing content that the current determination result $CurrLoopResult_i$ is a result obtained after the real-time working current is processed by a function $func\_ProcessCurr$ in the foregoing process. If the first current determination result or the second current determination result indicates an abnormality, whether the abnormality is corrected within a preset time step needs to be detected. If the abnormality can be corrected within the preset time step, the fault type is determined as the soft fault. Otherwise, if the abnormality is not corrected within the preset time step, the fault type is determined as the hard fault.

By combining the voltage determination result and the current determination result, whether the soft fault or the hard fault occurs in the physiological parameter detection apparatus can be accurately distinguished. However, by detecting whether the abnormality is corrected within the preset time step, the soft fault can be dynamically monitored and determined. This method not only considers instantaneous interference, but also considers a possibility of a persistent fault, thereby improving accuracy and flexibility of fault determination. By means of the foregoing accurate fault type determination and a timely fault processing, problems of instability and reduced reliability of the physiological parameter detection apparatus caused by a fault can be effectively reduced, thereby helping to ensure continuous and accurate operation of the physiological parameter detection apparatus.

Further, in an embodiment, as shown in FIG. 6, after determining the fault type of the physiological parameter detection apparatus, the method further includes: counting a number of times the physiological parameter detection apparatus has a hard fault, and outputting an alerting signal to the physiological parameter detection apparatus if the counted number of times exceeds a preset number threshold. A number of times of hard faults is counted, and when the number of times of faults reaches or exceeds a threshold, an alerting signal is output to remind maintenance personnel to perform checking and maintenance in time, thereby avoiding faults of a device at a critical time, and ensuring continuous and stable operation of the device. In addition, frequent hard faults may alternatively mean that the device has a serious quality problem or is about to reach a service life. Therefore, outputting the alerting signal in time may prompt relevant personnel to take measures, for example, replace the device or perform more in-depth technical maintenance, thereby reducing risks and losses caused by the fault of the physiological parameter detection apparatus.

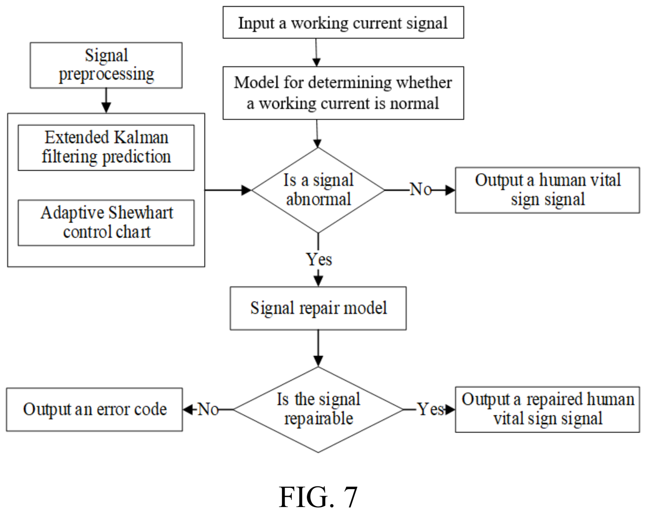

Optionally, as shown in Fig.7, step S104 of the method according to the first embodiment of this application may further incorporate the model for determining whether a working current is normal in the second embodiment. Specifically, if the determination result from the model for determining whether a working current is normal in the second embodiment indicates an abnormality, it may also be determined that the corresponding human vital sign signal exhibits an abnormality.

Optionally, the method further comprises:

determining whether the working parameter includes a real-time working voltage;
based on a determination that the working parameter includes the real-time working voltage, implementing a current-and-voltage-based anomaly detection process and
based on a determination that the working parameter does not include the real-time working voltage, implementing a current-based anomaly detection process.

Preferably, the current-and-voltage-based anomaly detection process comprises steps of the method according to the second embodiment (particularly the method for detecting a fault type of a physiological parameter detection apparatus) and the current-based anomaly detection process comprises steps of the method according to the first embodiment (particularly the method for detecting an error code in a human vital sign signal).

The method of detecting an anomaly of a signal from a physiological parameter detection apparatus described in the present application enables detecting an error code in a human vital sign signal and/or detecting a fault type of a physiological parameter detection apparatus.

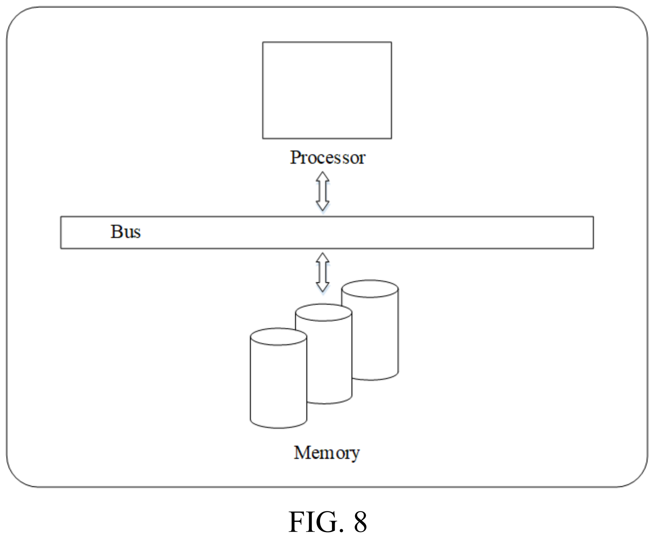

In another aspect, as shown in FIG. 8, an embodiment of this application further provides a device for detecting an anomaly of a signal from a physiological parameter detection apparatus (particularly a device for detecting an error code in a human vital sign signal or a device for detecting a fault type of a physiological parameter detection apparatus), comprising:

at least one processor; and
a memory communicatively connected to the at least one processor,
where the memory has instructions that are executable by the at least one processor stored therein, and the instructions are executed by the at least one processor to cause the at least one processor to perform the method for detecting the error code in the human vital sign signal described in any one of the above embodiments.

An embodiment of this application further provides a non-volatile computer storage medium, having computer-executable instructions stored therein. The computer-executable instructions are configured to implement the method of detecting an anomaly of a signal from a physiological parameter detection apparatus described in any one of the above embodiments (particularly the method for detecting an error code in a human vital sign signal or the method for detecting a fault type of a physiological parameter detection apparatus).

An embodiment of this present application further provides a physiological parameter detection apparatus, comprising the aforementioned device for an anomaly of a signal from the physiological parameter detection apparatus.

The embodiments of this application are all described in a progressive manner, for same or similar parts in the embodiments, refer to such embodiments, and descriptions of each embodiment focus on a difference from other embodiments. Particularly, the device and medium embodiments are basically similar to the method embodiment, and therefore are described briefly. For related parts, refer to some of the descriptions in the method embodiment.

The device and the medium that are provided in the embodiments of this application are in a one-to-one correspondence with the method. Therefore, the device and the medium also have the beneficial technical effects similar to those of the method corresponding to the device and the medium. The beneficial technical effects of the method have been described in detail above, so that the beneficial technical effects of the device and the medium are not elaborated herein again.

A person skilled in the art can understand that the embodiments of this application may be provided as a method, a system, or a computer program product. Therefore, this application may use a form of hardware-only embodiments, software-only embodiments, or embodiments combining software and hardware. Moreover, this application may use a form of a computer program product that is implemented on one or more computer-usable storage media (including but not limited to a disk memory, a CD-ROM, an optical memory, and the like) that include computer-usable program code.

This application is described with reference to flowcharts and/or block diagrams of the method, the device (system), and the computer program product according to the embodiments of this application. It should be understood that computer program instructions can implement each procedure and/or block in the flowcharts and/or block diagrams and a combination of procedures and/or blocks in the flowcharts and/or block diagrams. These computer program instructions may be provided to a general-purpose computer, a special-purpose computer, an embedded processor, or a processor of another programmable data processing device to generate a machine, so that an apparatus configured to implement functions specified in one or more procedures in the flowcharts and/or one or more blocks in the block diagrams is generated by using instructions executed by the computer or the processor of another programmable data processing device.

These computer program instructions may alternatively be stored in a computer-readable memory that can instruct a computer or another programmable data processing device to work in a specific manner, so that the instructions stored in the computer-readable memory generate an artifact that includes an instruction apparatus. The instruction apparatus implements a specific function in one or more procedures in the flowcharts and/or in one or more blocks in the block diagrams.

These computer program instructions may further be loaded onto a computer or another programmable data processing device, so that a series of operations and steps are performed on the computer or the another programmable device, thereby generating computer-implemented processing. Therefore, the instructions executed on the computer or the another programmable device provide steps for implementing a specific function in one or more processes in the flowcharts and/or in one or more blocks in the block diagrams.

In a typical configuration, a computing device includes one or more central processing units (CPUs), an input/output interface, a network interface, and an internal memory.

The memory may include a form such as a volatile memory, a random-access memory (RAM) and/or a non-volatile memory such as a read-only memory (ROM) or a flash RAM in a computer-readable medium. The memory is an example of the computer-readable medium.

The computer-readable medium includes a non-volatile medium and a volatile medium, a removable medium and a non-removable medium, which may implement storage of information by using any method or technology. The information may be a computer-readable instruction, a data structure, a program module, or other data. Examples of a storage medium of a computer includes, but is not limited to, a phase-change memory (PRAM), a static random access memory (SRAM), a dynamic random access memory (DRAM), or other types of RAMs, a ROM, an erasable programmable read-only memory (EEPROM), a flash memory or another storage technology, a compact disc read-only memory (CD-ROM), a digital versatile disc (DVD) or another optical storage, a cartridge tape, a magnetic tape, a magnetic disk storage or another magnetic storage device, or any other non-transmission medium, which may be configured to store information accessible by a computing device. According to limitations of this specification, the computer-readable medium does not include transitory computer-readable media, such as a modulated data signal and a modulated carrier.

It should be further noted that the terms "include", "comprise", or any variants thereof are intended to cover a non-exclusive inclusion. Therefore, a process, method, article, or device that includes a series of elements not only includes such elements, but also includes other elements not specified expressly, or may include inherent elements of the process, method, article, or device. Unless otherwise specified, an element limited by "include a/an..." does not exclude other same elements existing in the process, the method, the article, or the device that includes the element.

The above content is only the embodiments of this application, but not intended to limit this application. For a person skilled in the art, various modifications and variations can be made to this application.

## Claims

1. A method of detecting an anomaly of a signal from a physiological parameter detection apparatus, particularly a continuous glucose monitor, CGM, **characterized by** comprising:

   acquiring a working parameter of the physiological parameter detection apparatus, wherein the working parameter comprises a working current;
   determining whether the signal from the physiological parameter detection apparatus is abnormal based on the working parameter; and
   outputting a detection result.

2. The method according to claim 1, **characterized in that**:

the signal from the physiological parameter detection apparatus is a human vital sign signal, and the human vital sign signal is calculated from the working current, preferably the human vital sign signal is a blood glucose signal, and the method further comprises:

generating a first state vector based on the human vital sign signal and a preset signal value;
determining an estimated vital sign signal at a current moment based on a human vital sign signal at a previous moment through a filtering model; determining a predicted signal interval at the current moment based on the estimated vital sign signal and a human vital sign signal within a preset time window through a constraint model; generating a second state vector based on the estimated vital sign signal and the predicted signal interval; and determining, based on the first state vector and the second state vector, whether an error code exists in a human vital sign signal at the current moment, and outputting the detection result.

3. The method according to claim 2, **characterized in that** the determining an estimated vital sign signal at a current moment based on a human vital sign signal at a previous moment through a filtering model comprises:

linearizing a state equation of the filtering model to obtain a linear state equation; and
obtaining the estimated vital sign signal at the current moment based on an optimal estimation result of the human vital sign signal at the previous moment by using the linear state equation.

4. The method according to claim 2 or 3, **characterized in that** the determining a predicted signal interval at the current moment based on the estimated vital sign signal and a human vital sign signal within a preset time window through a constraint model comprises:

calculating a signal standard deviation and a signal mean based on the human vital sign signal within the preset time window; and
inputting the signal standard deviation, the signal mean, the human vital sign signal at the previous moment, and the estimated vital sign signal to the constraint model, and determining, by the constraint model, the predicted signal interval at the current moment;
preferably, wherein the predicted signal interval at the current moment comprises a maximum critical value and a minimum critical value; a calculation formula of the maximum critical value is:

$$\mathrm{upLimit\_CgmSignal_i}$$
$$= G(\mathrm{Pro\_CgmSignal_{i-1}}, \mathrm{Ave\_Pro\_CgmSignal_N}, \bar{x}_{k+1|k}) + \gamma$$
$$* \mathrm{Std\_Pro\_CgmSignal_N}$$

a calculation formula of the minimum critical value is:

$$\mathrm{LowLimit\_CgmSignal_i}$$
$$= G(\mathrm{Pro\_CgmSignal_{i-1}}, \mathrm{Ave\_Pro\_CgmSignal_N}, \bar{x}_{k+1|k}) - \beta$$
$$* \mathrm{Std\_Pro\_CgmSignal_N}$$

wherein $\mathrm{Pro\_CgmSignal_{i-1}}$ represents the human vital sign signal at the previous moment; $\mathrm{Ave\_Pro\_CgmSignal_N}$ represents the signal mean; $\mathrm{Std\_Pro\_CgmSignal_N}$ represents the signal standard deviation; and $\gamma$ and $\beta$ represent preset coefficients.

5. The method according to any one of claims 2 to 4, **characterized in that** the preset signal value comprises a first signal value, a second signal value, and a third signal value; before the generating a first state vector based on a human vital sign signal and a preset signal value, the method further comprises:

preprocessing the human vital sign signal based on the first signal value, the second signal value, and the third signal value, to obtain a preprocessed vital sign update signal; and
correspondingly, the generating a first state vector based on a human vital sign signal and a preset signal value comprises:

generating the first state vector based on the vital sign update signal;

preferably, wherein the preprocessing the human vital sign signal based on the first signal value, the second signal value, and the third signal value comprises:

updating the human vital sign signal to the first signal value if the human vital sign signal is greater than the first signal value;

updating the human vital sign signal to a sum value of the human vital sign signal at the previous moment and the second signal value if the human vital sign signal is greater than the human vital sign signal at the previous moment and a difference between the human vital sign signal and a human vital sign signal at the previous moment is greater than the second signal value; and

updating the human vital sign signal to a difference between the human vital sign signal at the previous moment and the third signal value if the human vital sign signal is less than the human vital sign signal at the previous moment and an absolute value of the difference between the human vital sign signal and the human vital sign signal

at the previous moment is greater than the third signal value; and/or preferably, wherein the generating the first state vector based on the vital sign update signal comprises:

determining a state value corresponding to the vital sign update signal as a first preset value if the human vital sign signal is greater than or equal to the first signal value or if the human vital sign signal is 0;

determining the state value corresponding to the vital sign update signal as the first preset value if the human vital sign signal is greater than a human vital sign signal at the previous moment and an absolute value of the difference between the human vital sign signal and the human vital sign signal at the previous moment is greater than or equal to the second signal value;

determining the state value corresponding to the vital sign update signal as the first preset value if the human vital sign signal is less than a human vital sign signal at the previous moment and the absolute value of the difference between the human vital sign signal and the human vital sign signal at the previous moment is greater than or equal to the third signal value;

determining the state value corresponding to the vital sign update signal as a second preset value if the human vital sign signal is not less than a human vital sign signal at the previous moment, or the absolute value of the difference between the human vital sign signal and the human vital sign signal at the previous moment is less than the third signal value; and

generating the first state vector based on the state value of each vital sign update signal.

6. The method according to any one of claims 2 to 5, **characterized in that** the generating a second state vector based on the estimated vital sign signal and the predicted signal interval comprises:

determining a state value corresponding to the estimated vital sign signal as a second preset value if the estimated vital sign signal falls within the predicted signal interval;

determining the state value corresponding to the estimated vital sign signal as a first preset value if the estimated vital sign signal does not fall within the predicted signal interval; and

generating the second state vector based on the state value of each estimated vital sign signal;

and/or

the determining, based on the first state vector and the second state vector, whether an error code exists in a human vital sign signal at the current moment comprises:

if a state value of the first state vector corresponding to the estimated vital sign signal is a first preset value or a state value of the second state vector is the first preset value, determining that an error code exists in the human vital sign signal at the current moment; and

if the state value of the first state vector corresponding to the estimated vital sign signal is a second preset value and the state value of the second state vector is the second preset value, determining that the human vital sign signal at the current moment is a valid code;

preferably wherein after the determining, based on the first state vector and the second state vector, whether an error code exists in a human vital sign signal at the current moment, the method further comprises:

determining an optimal estimation result corresponding to the human vital sign signal at the current moment, and acquiring a number of consecutive occurrences of determining that the human vital sign signal at the current moment is a valid code;

outputting the human vital sign signal at the current moment as an unrepairable signal if the number of consecutive occurrences is greater than or equal to a third preset value; and

outputting the human vital sign signal at the current moment as a repairable signal if the number of consecutive occurrences is less than the third preset value.

7. The method according to claim 1, **characterized in that** the working parameter further comprises a working voltage.

8. The method according to claim 7, **characterized in that** the working voltage comprises a real-time working voltage, the working current comprises a real-time working current, and the method further comprises:

comparing the real-time working voltage with a first preset threshold to obtain a voltage determination result, and comparing the real-time working current with a second preset threshold to obtain a current determination result; and

determining whether the signal from the physiological parameter detection apparatus is abnormal, wherein the signal from the physiological parameter detection apparatus is the real-time working current.

9. The method according to claim 8, **characterized in that** the method further comprises:
determining a fault type of the physiological parameter detection apparatus according to the voltage determination result and the current determination result.

10. The method according to claim 9, **characterized in that** the real-time working voltage comprises a working electrode voltage, a reference electrode voltage, and a counter electrode voltage, the first preset threshold comprises a first preset voltage threshold and a second preset voltage threshold, and the comparing the real-time working voltage with a first preset threshold to determine a voltage determination result comprises:

comparing the working electrode voltage with the first preset voltage threshold, and comparing the reference electrode voltage with the second preset voltage threshold, to determine a first voltage determination result;

determining a second voltage determination result based on the working electrode voltage, the reference electrode voltage, and the counter electrode voltage; and

determining that the voltage determination result indicates an abnormality if there is an abnormal result in the first voltage determination result or the second voltage determination result;

preferably, wherein the first preset threshold further comprises a first preset voltage difference threshold and a second preset voltage difference threshold, and the determining a second voltage determination result based on the working electrode voltage, the reference electrode voltage, and the counter electrode voltage comprises:

determining a first voltage difference between the working electrode voltage and the reference electrode voltage, and a second voltage difference between the working electrode voltage and the counter electrode voltage; and

determining that the second voltage determination result indicates an abnormality if the first voltage difference is greater than the first preset voltage difference threshold or the second voltage difference is greater than the second preset voltage difference threshold.

11. The method according to claim 9 or 10, **characterized in that** the comparing the real-time working current with a second preset threshold to determine a current determination result comprises:

obtaining a first scope value corresponding to detection sensitivity of the physiological parameter detection apparatus, and obtaining a second scope value corresponding to a to-be-detected physiological parameter;

determining a threshold interval of the real-time working current according to the first scope value and the second scope value, and determining the real-time working current based on the threshold interval, to obtain a first current determination result;

performing signal filtering processing on the real-time working current, and determining a second current determination result according to the processed real-time working current and a preset current threshold;

performing low-pass filtering processing on the real-time working current to obtain a baseline of the real-time working current, and determining a third current determination result based on drift data of the baseline and a corresponding preset drift data threshold; and

determining that the current determination result indicates an abnormality if there is an abnormal result in the first current determination result, the second current determination result, or the third current determination result;

preferably, wherein the fault type comprises a soft fault and a hard fault, and the determining a fault type of the physiological parameter detection apparatus according to the voltage determination result and the current determination result comprises:

determining that the fault type is the hard fault if the voltage determination result is an abnormality;

determining that the fault type is the hard fault if the third current determination result is an abnormality; and

detecting whether the abnormality is corrected within a preset time step if the first current determination result or the second current determination result is an abnormality; determining that the fault type is the soft fault if the abnormality is corrected within the preset time step; and determining that the fault type is the hard fault if the abnormality is not corrected within the preset time step;

preferably, wherein after the determining a fault type of the physiological parameter detection apparatus, the method further comprises:

counting a number of times the physiological parameter detection apparatus has a hard fault, and outputting, by the physiological parameter detection apparatus, an alarm signal if the counted number of times exceeds a preset number threshold.

12. The method according to any one of claims 9 to 11, **characterized in that** before the obtaining a real-time working voltage and a real-time working current of the physiological parameter detection apparatus, the method further comprises:

collecting an initial voltage of the physiological parameter detection apparatus according to a preset sampling period;

calculating an average voltage, a standard deviation, and a range value that correspond to the initial voltage, and determining whether the average voltage, the standard deviation, and the range value are all within corresponding preset thresholds; and

if the average voltage, the standard deviation, and the range value are all within the corresponding preset thresholds, obtaining the real-time working voltage and the real-time working current of the physiological parameter detection apparatus;

and/or

the method further comprises:

determining that the real-time working current is an invalid signal if the voltage determination result or the current determination result is an abnormality.

13. The method according to claim 1, **characterized in that** the method further comprises:

determining whether the working parameter includes a real-time working voltage;

based on a determination that the working parameter includes the real-time working voltage, implementing a current-and-voltage-based anomaly detection process, preferably the current-and-voltage-based anomaly detection process comprises steps of the method according to any one of claims 8 to 12; and

based on a determination that the working parameter does not include the real-time working voltage, implementing a current-based anomaly detection process, preferably the current-based anomaly detection process comprises steps of the method according to any one of claims 1 to 6;

14. A device for detecting an anomaly of a signal from a physiological parameter detection apparatus, particularly a continuous glucose monitor, CGM, **characterized by** comprising:

at least one processor; and

a memory communicatively connected to the at least one processor,

wherein the memory has instructions that are executable by the at least one processor stored therein, and the instructions are executed by the at least one processor to cause the at least one processor to perform the method for detecting an anomaly of a signal from a physiological parameter detection apparatus according to any one of claims 1 to 13.

15. A non-volatile computer storage medium, having computer-executable instructions stored therein, wherein the computer-executable instructions are configured to implement the method of an anomaly of a signal from a physiological parameter detection apparatus according to any one of claims 1 to 13.

S1

acquiring a working parameter of the physiological parameter detection apparatus, wherein the working parameter comprises a working current

S2

based on the working parameter, determining whether the signal from the physiological parameter detection apparatus is abnormal

S3

outputting a detection result

FIG. 1

S101

Generate a first state vector based on a human vital sign signal and a preset signal value

S102

Determine an estimated vital sign signal at a current moment based on a human vital sign signal at a previous moment through a filtering model, and determine a predicted signal interval at the current moment based on the estimated vital sign signal and a human vital sign signal within a preset time window through a constraint model

S103

Generate a second state vector based on the estimated vital sign signal and the predicted signal interval

S104

Determine, based on the first state vector and the second state vector, whether an error code exists in a human vital sign signal at the current moment, and output a detection result

FIG. 2

FIG. 3

Obtain a real-time working voltage and a real-time working current of a physiological parameter detection apparatus — S201

Compare the real-time working voltage with a first preset threshold to obtain a voltage determination result, and compare the real-time working current with a second preset threshold to obtain a current determination result — S202

Determine a fault type of the physiological parameter detection apparatus according to the voltage determination result and the current determination result — S203

FIG. 4

$$V_{OUT} = V_B + V_M + I_S \times R_S$$

FIG. 5

Input a working electrode
voltage signal

Input a counter electrode
voltage signal

Input a reference
electrode voltage signal

Model for determining
whether a working voltage
is normal

Input a working current
signal

Model for determining
whether a working current
is normal

Output a working
current signal

Yes

Whether
the signal is
valid

No

A current signal is not output

Determine a fault type

The fault type is
hard fault

The fault type
is soft fault

A frequency of
hard fault is
accumulated

Whether a
frequency of
faults exceeds a
threshold

Yes

Early
apparatus
alert

No

FIG. 6

FIG. 7

FIG. 8

Europäisches
Patentamt
European
Patent Office
Office européen
des brevets

# EUROPEAN SEARCH REPORT

**Application Number**

EP 25 22 7423

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 113 365 555 A (MEDTRONIC MINIMED INC) 7 September 2021 (2021-09-07) * paragraphs [0009], [0192], [0291], [0197], [0254], [0346] * * paragraphs [0349], [0406], [0858], [0953], [0974], [1140] - [1142], [1200] * ----- | 1-15 | INV. G16H40/40 G16H40/63 |

TECHNICAL FIELDS
SEARCHED     (IPC)

G16H

The present search report has been drawn up for all claims

| Place of search | Date of completion of the search | Examiner |
|---|---|---|
| Munich | 28 April 2026 | Samulowitz, Michael |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 22 7423

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2026

| Patent document cited in search report | Publication date | Patent family member(s) | Publication date |
|---|---|---|---|
| CN 113365555 A | 07-09-2021 | CN 113365555 A | 07-09-2021 |
| | | CN 120167953 A | 20-06-2025 |
| | | EP 3917395 A1 | 08-12-2021 |
| | | US 2020245910 A1 | 06-08-2020 |
| | | US 2024423514 A1 | 26-12-2024 |
| | | WO 2020160023 A1 | 06-08-2020 |

EPO FORM P0459

For more details about this annex : see Official Journal of the European Patent Office, No. 12/82